# EUROPEAN PATENT APPLICATION

(11) **EP 4 767 923 A1**
(43) Date of publication of application: **01.07.2026**
(21) Application number: 24886171.8
(22) Date of filing: 28.10.2024
(51) Int. Cl.: A61B 3/113, A61B 3/00

(54) **METHOD AND APPARATUS FOR GENERATING OCULAR MOVEMENT ABILITY INFORMATION**

(30) Priority: 30.10.2023 KR 20230147215
(71) Applicant: Korea University Research and Business Foundation, Seoul 02841 (KR)
(72) Inventor: SUH, Young Woo, Seoul 06374 (KR); HWANG, In Tae, Ansan-si, Gyeonggi-do 15355 (KR)
(74) Representative: IPAZ
(86) International application number: PCT/KR2024/016511
(87) International publication number: WO 2025/095497

(57) **Abstract**

In the present specification, a method and an apparatus for generating ocular movement ability information are disclosed. The method for generating ocular movement ability information, according to the present specification, is a method for generating ocular movement ability information using a device including an RGB camera, a depth sensor, and an ocular tracking sensor in a housing of a predetermined shape mounted on a head of a subject, and may comprise: (a) calculating, by a processor, coordinates for a plurality of grid points in image data obtained by photographing an ocular movement indicator input from the RGB camera; (b) receiving, from the ocular tracking sensor by the processor, a measurement value for the gaze direction of one of the eyes of the subject of which the field of view is blocked, and storing the measurement value; (c) storing, by the processor, timing (hereinafter, referred to as "timing information") at which one of the eyes of the subject of which the field of view is open fixates at the grid points included in the ocular movement indicator; and (d) calculating, by the processor, a gaze error of the subject with respect to each of the grid points by using distance information between the subject and the indicator input from the depth sensor, the timing information, and gaze tracking information.

## Description

### [Technical Field]

The present disclosure relates to a method and apparatus for generating ocular movement ability information, and more particularly, to a method and an apparatus for generating ocular movement ability information using augmented reality equipment.

### [Background Art]

This application claims the priority of Korean Patent Application No. 10-2023-0147215 filed on October 30, 2023, in the Korean Intellectual Property Office, the disclosure of which is incorporated herein by reference.

The contents described in this part merely provide background information on the exemplary embodiment of the present specification, but do not constitute the related art.

In order to inspect the movement ability of eyeballs of a subject, the Hess chart test is conducted. During the Hess chart test, a subject fixates at a test chart while wearing red-green glasses and fixing a face. A tester provides a target by projecting a red light pointer on a test point on the chart and the subject projects the target with a green light pointer. Next, the tester records a point projected by the subject with the pointer on a result sheet. For the measurement of the opposite eye, the tester and the subject exchange their pointers. However, according to the Hess chart test of the related art, an eyeball of the subject is not tracked and an error may occur due to a minute head movement during the process of recording the point projected by the tester with the pointer on the result sheet and the test process.

Recently, along with the virtual environment technology, a technology for measuring an ocular movement ability using virtual reality equipment has been developed. A subject wears the VR equipment and looks at a virtual test chart provided on a display device of the VR equipment. However, due to the limited distance from the display device, there is a disadvantage of narrowing a viewing angle of a subject who fixates at the virtual test chart.

### [Disclosure]

### [Technical Problem]

An object of the present specification is to provide an apparatus and a method for generating ocular movement ability information.

Objects of the present specification are not limited to the above-mentioned objects, and other objects, which are not mentioned above, can be clearly understood by those skilled in the art from the following descriptions.

### [Technical Solution]

In order to achieve the above-described object, a method for generating ocular movement ability information, according to the present specification, is a method for generating ocular movement ability information using a device including an RGB camera, a depth sensor, and an ocular tracking sensor in a housing of a predetermined shape mounted on a head of a subject, and may include: (a) calculating, by a processor, coordinates for a plurality of grid points in image data obtained by photographing an ocular movement indicator input from the RGB camera; (b) receiving, from the ocular tracking sensor by the processor, a measurement value for the gaze direction of one of the eyes of the subject of which the field of view is blocked, and storing the measurement value; (c) storing, by the processor, timing (hereinafter, referred to as "timing information") at which one of the eyes of the subject of which the field of view is open fixates at the grid points included in the ocular movement indicator; and (d) calculating, by the processor, a gaze error of the subject with respect to each of the grid points by using distance information between the subject and the indicator input from the depth sensor, the timing information, and the gaze tracking information.

According to the exemplary embodiment of the present specification, the housing further includes a microphone which receives an audio signal, and wherein the (c) is, receiving, by the processor, an audio signal from the microphone or information of a target image presented at the grid point from the RGB camera to store the timing information.

According to the exemplary embodiment of the present specification, the housing further includes a field-of-view blocking device which individually blocks fields of view of two eyes of the subject and wherein the (b), the processor outputs a blocking signal to the field-of-view blocking device to block a field of view of one eye of the subject.

According to the exemplary embodiment of the present specification, wherein the (d) is, quantifying, by the processor, at least one of an average distance error between gaze positions of the subject in the grid points, a direction error for each grid point, and an total error.

The method for generating ocular movement ability information according to the present specification may further include, after the (d), (e) outputting, by the processor, a graph for a position of a gaze of the subject according to each grid point.

The method for generating movement ability information according to the present specification may be implemented as a computer program which is created to perform each step in the computer to be recorded in a computer readable recording medium.

An apparatus for generating ocular movement ability information according to the present specification may include, a predetermined shape of housing which is mounted on a head of a subject and includes an RGB camera, a depth sensor, and an ocular tracking sensor; a coordinate calculation processor which calculates coordinates for a plurality of grid points in image data obtained by photographing an ocular movement indicator input from the RGB camera; a gaze information storage processor which receives a measurement value for a gaze direction of one of the eyes of the subject of which the field of view is blocked and stores the measurement value; a timing storage processor which stores timing (hereinafter, referred to as "timing information") at which one of the eyes of the subject of which the field of view is open fixates at the grid points included in the ocular movement indicator; and a movement ability calculation processor which calculates a gaze error of the subject with respect to each of the grid points by using distance information between the subject and the indicator input from the depth sensor, the timing information, and the gaze tracking information.

According to the exemplary embodiment of the present specification, the housing further includes a microphone which receives an audio signal, and the timing storage processor receives an audio signal from the microphone or information of a target image presented at the grid point from the RGB camera to store the timing information.

According to the exemplary embodiment of the present specification, the housing further includes a field-of-view blocking device which individually blocks fields of view of two eyes of the subject and the gaze information storage processor outputs a blocking signal to the field-of-view blocking device to block a field of view of one eye of the subject.

According to the exemplary embodiment of the present specification, the movement ability calculation processor may quantify at least one of an average distance error between gaze positions of the subject in the grid points, a direction error for each grid point, and an total error.

The apparatus for generating ocular movement ability information according to the present specification may further include a graph output processor which outputs a graph for a position of a gaze of the subject according to each grid point.

The apparatus for generating ocular movement ability information according to the present specification may be one component of a system for generating ocular movement ability information including: an ocular movement indicator on which a plurality of grid points is drawn; a target generation device which presents a target image on the grid point; a computing device which outputs an actuation signal of the apparatus for generating ocular movement ability information and receives ocular movement ability information; and a communication device which transmits and receives a signal or information between the apparatus for generating ocular movement ability information, the computing device, and the target generation device.

Other detailed matters of the present disclosure are included in the detailed description and the drawings.

### [Advantageous Effects]

According to an aspect of the present specification, an eyeball of a subject may be tracked using augmented reality equipment more accurately than the related art.

According to another aspect of the present specification, an error which occurs during the process of moving a point projected by a subject with a laser pointer to a result sheet by a tester in the related art may be reduced.

According to another aspect of the present specification, a problem of narrowing a field of view which is caused during the ocular test using the VR equipment of the related art may be solved.

The effects of the present disclosure are not limited to the technical effects mentioned above, and other effects which are not mentioned can be clearly understood by those skilled in the art from the following description

### [Description of Drawings]

FIG. 1 is a block diagram of an ocular movement ability information generation apparatus according to an exemplary embodiment of the present specification.
FIG. 2 is an exemplary image for conducting an ocular movement ability test.
FIG. 3 is a block diagram of an ocular movement ability information generation apparatus according to another exemplary embodiment of the present specification.
FIG. 4 is a block diagram of an ocular movement ability information generation apparatus according to still another exemplary embodiment of the present specification.
FIG. 5 is an exemplary image for an item quantified to evaluate an ocular movement ability according to an exemplary embodiment of the present specification.
FIG. 6 is a block diagram of an ocular movement ability information generation apparatus according to still another exemplary embodiment of the present specification.
FIG. 7 is an exemplary image of a graph according to gaze positions of a left eye and a right eye of a subject.
FIG. 8 is a schematic image of an ocular movement ability information generation system according to an exemplary embodiment of the present specification.
FIG. 9 is an exemplary image illustrating linkage between an ocular movement ability information generation apparatus and a computing device.
FIG. 10 is a flowchart of an ocular movement ability information generation method according to an exemplary embodiment of the present specification.
FIG. 11 is a flowchart of an ocular movement ability information generation method according to another exemplary embodiment of the present specification.
FIG. 12 is a flowchart of an ocular movement ability information generation method according to still another exemplary embodiment of the present specification.
FIG. 13 is a flowchart of an ocular movement ability information generation method according to still another exemplary embodiment of the present specification.
FIG. 14 is a flowchart of an ocular movement ability information generation method according to still another exemplary embodiment of the present specification.

### [Best Mode]

Advantages and characteristics of the present disclosure disclosed in the present specification, and a method of achieving the advantages and characteristics will be clear by referring to exemplary embodiments described below in detail together with the accompanying drawings. However, the present specification is not limited to exemplary embodiments disclosed herein but will be implemented in various different forms. The exemplary embodiments are provided by way of example only so that this disclosure will be thorough and complete, and a person of ordinary skill in the art can fully understand the disclosures of the present specification and the scope of the present specification. Therefore, the present specification will be defined only by the scope of the appended claims.

The terms used in the present specification are for explaining the embodiments rather than limiting the scope of the present specification. Unless particularly stated otherwise in the present specification, a singular form also includes a plural form. The term "comprise" and/or "comprising" used in the specification does not exclude the presence or addition of one or more other components in addition to the mentioned component.

Like reference numerals generally denote like elements throughout the specification and "and/or" includes each of mentioned components and all combinations of one or more components. Although the terms "first", "second", and the like are used for describing various components, these components are not confined by these terms. These terms are merely used for distinguishing one component from the other components. Therefore, a first component to be mentioned below may be a second component in a technical spirit of the present disclosure.

Unless otherwise defined, all terms (including technical and scientific terms) used in the present specification may be used as the meaning which may be commonly understood by the person with ordinary skill in the art, to which the present disclosure belongs. It will be further understood that terms defined in commonly used dictionaries should not be interpreted in an idealized or excessive sense unless expressly and specifically defined.

Hereinafter, exemplary embodiments of the present specification will be described in detail with reference to the accompanying drawings.

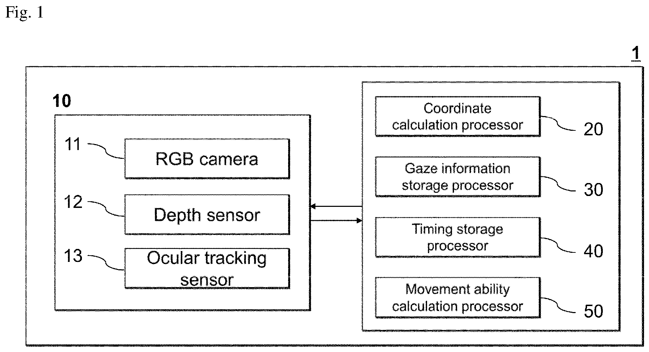

FIG. 1 is a block diagram of an ocular movement ability information generation apparatus according to an exemplary embodiment of the present specification.

Referring to FIG. 1, an ocular movement ability information generation apparatus 1 according to an exemplary embodiment of the present specification may include a housing 10, a coordinate calculation processor 20, a gaze information storage processor 30, a timing storage processor 40, and a movement ability calculation processor 50.

The housing 10 may be mounted on a head of a subject. The housing 10 is a head-mounted display (HMD) of augmented reality equipment. The housing 10 may be any one of smart glasses, smart goggles, or helmet-mounted display, which is an example, but is not limited by a specific shape.

The housing 10 may include an RGB camera 11, a depth sensor 12, and an ocular tracking sensor 13. The RGB camera 11 may capture an ocular movement ability test indicator to store it as image data. The depth sensor 12 may output distance information from the subject equipped with the housing 10 to the indicator. The ocular tracking sensor 13 may track a direction of the eyeball of the subject when the subject looks at a target presented on the indicator. The ocular tracking sensor 13 may be an infrared camera, which is just an example, but is not limited by specific equipment.

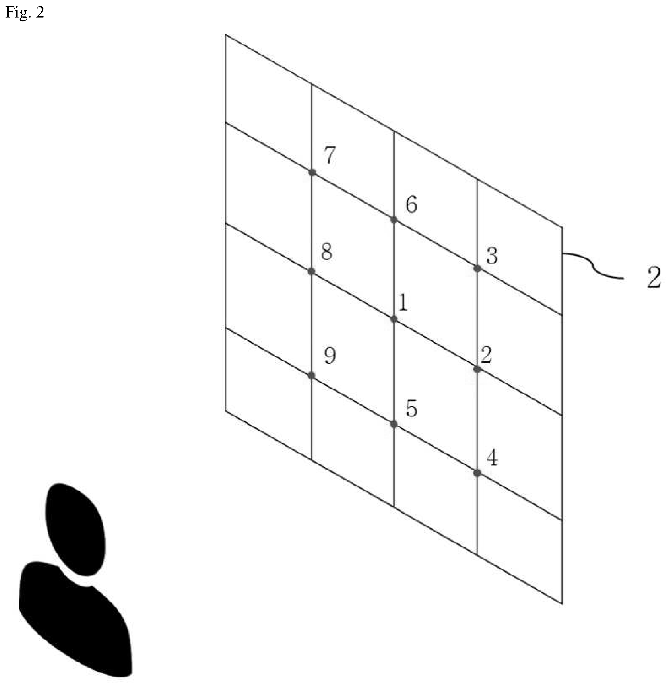

FIG. 2 is an exemplary image for conducting an ocular movement ability test.

Referring to FIG. 2, the subject equipped with the housing 10 looks at the indicator 2. The indicator 2 may be any one of a Hess screen chart, a Harms screen chart, and/or a Lancaster screen chart, which corresponds to one example, but is not limited by a specific indicator.

The RGB camera 11 may capture the indicator 2 to store it as image data. The coordinate calculation processor 20 may receive image data of the indicator 2 from the RGB camera 11 to calculate coordinates for a plurality of grid points in the image of the indicator 2.

In order to calculate the coordinates, line detection, grid search, edge detection algorithms and/or the deep learning model may be used, which are just examples, but are not limited by a specific method.

In order to conduct the ocular movement ability test, a tester may present a target at any one of the grid points to allow the subject to fixate at the target.

According to one exemplary embodiment of the present specification, the tester may present the target at the grid points of the indicator 2 in a predetermined order. The target may be presented at a center point (hereinafter, "first grid point") of the indicator 2 first. Next, the target may be presented at a right grid point (hereinafter, "second grid point") of the center point. Next, the target may be presented at a right upper grid point (hereinafter, "third grid point") of the center point. Next, the target may be presented at a right lower grid point (hereinafter, "fourth grid point") of the center point. Next, the target may be presented at a lower grid point (hereinafter, "fifth grid point") of the center point. Next, the target may be presented at an upper point (hereinafter, "sixth grid point") of the center point. Next, the target may be presented at a left upper grid point (hereinafter, "seventh grid point") of the center point. Next, the target may be presented at a left grid point (hereinafter, "eighth grid point") of the center point. Next, the target may be presented at a left lower grid point (hereinafter, "ninth grid point") of the center point. This corresponds to an example, but is not limited by the order.

The target may be presented on the indicator 2 by the tester using a laser pointer. Alternatively, the tester may verbally command the subject to fixate at a specific grid point. Alternatively, the indicator 2 may include a light emitting device at the grid points and light may be emitted at each grid point in a predetermined order. Alternatively, a target may be generated at a specific grid point using a target generation device. The target generation device may be an optical device which projects a specific marker to each grid point. This corresponds to an example, but is not limited by a specific method.

The subject may sequentially fixate at a target presented at the grid point while covering the field of view of one eyeball of two eyes. The subject may fixate at the target for a predetermined time. At this time, the ocular tracking sensor 13 may track a gaze direction of the subject to calculate a measurement value for the gaze direction. The gaze information storage processor 30 may store the measurement value for each grid point. At this time, the gaze information storage processor 30 may store a measurement value for a gaze direction of an eyeball having a closed field of view, between two eyes of the subject. If the test for one eye is completed, the subject may store a measurement value for the opposite eye.

When the subject fixates at the target, the timing storage processor 40 may store a timing (hereinafter, "timing information") at which the subject fixates at the target with an eyeball having an open field of view.

The movement ability calculation processor 50 may receive the timing information and the gaze information measurement value. Further, the movement ability calculation processor 50 may receive distance information between the subject and the indicator 2 from the depth sensor 12. The movement ability calculation processor 50 may calculate gaze information measurement value for each grid point corresponding to a specific timing using the timing information and calculate a gaze error of the subject using the distance information.

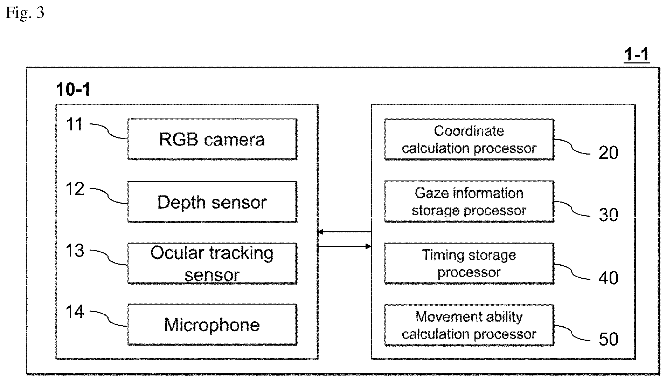

FIG. 3 is a block diagram of an ocular movement ability information generation apparatus according to another exemplary embodiment of the present specification.

Referring to FIG. 3, an ocular movement ability information generation apparatus 1-1 according to another exemplary embodiment of the present specification may include a housing 10-1 including an RGB camera 11, a depth sensor 12, an ocular tracking sensor 13, and a microphone 14, a coordinate calculation processor 20, a gaze information storage processor 30, a timing storage processor 40, and a movement ability calculation processor 50. The RGB camera 11, the depth sensor 12, the ocular tracking sensor 13, the coordinate calculation processor 20, the gaze information storage processor 30, the timing storage processor 40, and the movement ability calculation processor 50 have been described above so that a redundant description will be omitted.

The housing 10-1 may receive an audio signal through the microphone 14. The timing storage processor 40 may store the timing information using the audio signal or image information received from the RGB camera 11.

For example, the tester may verbally command the subject to fixate at the first grid point. At this time, the microphone 14 may receive the command to output the audio signal to the timing storage processor 40. If the timing storage processor 40 receives the audio signal, the timing storage processor may start recording timing information about the first grid point. Thereafter, the tester may verbally command the subject to fixate at the second grid point. If the timing storage processor 40 receives the audio signal to fixate at the second grid point, the timing storage processor may end recording the timing information about the first grid point and start recording timing information about the second grid point. Timing information about the third grid point to the ninth grid point is stored in the same way so that the redundant description will be omitted.

Next, the tester may command the subject to "cover an opposite eye". If the timing storage processor 40 receives the audio signal, the timing storage processor may start recording timing information about the opposite eye. Thereafter, timing information about the first grid point to the ninth grid point is stored in the same way so that the redundant description will be omitted.

The timing storage processor 40 may use a speech to text (STT) technique to analyze information about a voice from the input audio signal, which corresponds to an example, but is not limited by a specific technique.

Further, the tester may notify the subject in advance of a gazing order of grid points. Next, if the subject hears a beep sound, the tester may command the subject to sequentially fixate at the grid points. Next, the subject may fixate at the first grid point to the ninth grid point according to the beep sound. The timing storage processor 40 may receive a beep sound signal to store the timing information. This corresponds to an example, but is not limited by the test method.

As another example, the timing storage processor 40 may receive information of the target image presented at the first grid point to the ninth grid point from the RGB camera 11. If the the timing storage processor receives an image in which the target is presented at the first grid point, the timing storage processor 40 may start recording timing information about the first grid point. Next, if the the timing storage processor receives an image in which the target is presented at the second grid point, the timing storage processor 40 may end recording the timing information about the first grid point and start recording the timing information about the second grid point. Timing information about the third grid point to the ninth grid point is stored in the same way so that the redundant description will be omitted.

If the target is presented at the first grid point again, the timing storage processor 40 may start recording timing information about the opposite eye. Next, timing information about the first grid point to the ninth grid point is stored in the same way so that the redundant description will be omitted.

In order to analyze the presentation of the target at the grid point, the timing storage processor 40 may use image segmentation, template matching, and/or feature extraction techniques, which corresponds to an example, but is not limited by the above-described method.

Further, the timing storage processor 40 may receive images in which the target is presented at the first grid point to the ninth grid point, from the RGB camera 11 to store timing information about individual grid points. Next, the timing storage processor 40 may receive a voice signal of the tester, such as "cover the opposite eye", from the microphone 14 to store timing information about the opposite eye.

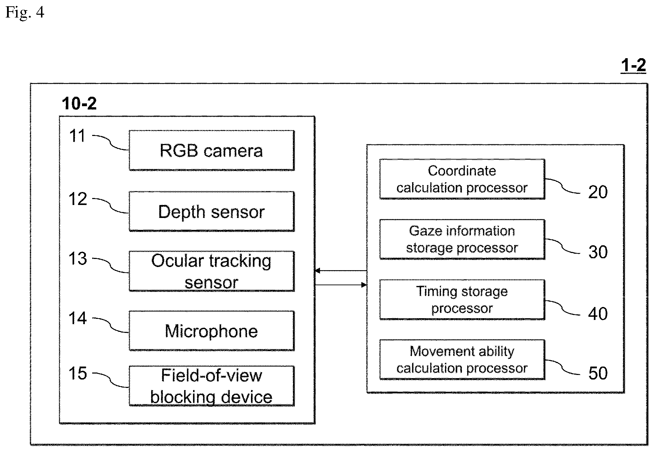

FIG. 4 is a block diagram of an ocular movement ability information generation apparatus according to still another exemplary embodiment of the present specification.

Referring to FIG. 4, an ocular movement ability information generation apparatus 1-2 according to still another exemplary embodiment of the present specification may include a housing 10-2 including an RGB camera 11, a depth sensor 12, an ocular tracking sensor 13, a microphone 14, and a field-of-view blocking device 15, a coordinate calculation processor 20, a gaze information storage processor 30, a timing storage processor 40, and a movement ability calculation processor 50. The RGB camera 11, the depth sensor 12, the ocular tracking sensor 13, the microphone 14, the coordinate calculation processor 20, the gaze information storage processor 30, the timing storage processor 40, and the movement ability calculation processor 50 have been described above so that a redundant description will be omitted.

The housing 10-2 may further include a field-of-view blocking device 15 which individually blocks fields of view of both eyes of the subject. The gaze information storage processor 30 may output a signal to the field-of-view blocking device to block a field of view of one eye of the two eyes of the subject. If the signal is input, the field-of-view blocking device 15 may block the field of view of the corresponding eyeball.

The gaze information storage processor 30 may receive image information of the target presented at the first grid point to the ninth grid point from the RGB camera 11. The gaze information storage processor 30 may receive an image in which the target is presented at the ninth grid point from the RGB camera 11. Next, the gaze information storage processor 30 may receive an image that the target disappears in the ninth grid point, from the RGB camera 11. At this time, the gaze information storage processor 30 may output a signal to the field-of-view blocking device 15 to unblock the field of view of the eyeball having a closed field of view and block a field of view of an opposite eyeball.

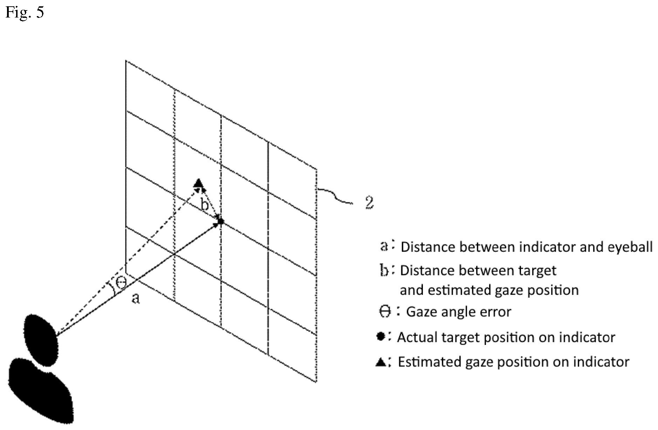

FIG. 5 is an exemplary image for an item quantified to evaluate an ocular movement ability according to an exemplary embodiment of the present specification.

Referring to FIG. 5, the movement ability calculation processor 50 may receive distance information (denoted by a of FIG. 5) between the indicator 2 and the eyeball from the depth sensor 12. The movement ability calculation processor 50 may receive the timing information and the gaze direction measurement value. The movement ability calculation processor 50 may calculate a mean value of the gaze direction using a gaze direction measurement value measured while the subject fixates at the target. The movement ability calculation processor 50 may calculate a coordinate of an estimated position of the gaze of the subject on the indicator 2 using the mean value. The movement ability calculation processor 50 may calculate a distance error (denoted by b in FIG. 5) between the estimated position and the target.

Further, the movement ability calculation processor 50 may calculate a direction error (denoted by θ in FIG. 5) between the estimated position and the grid point using the distance information.

Further, the movement ability calculation processor 50 may calculate a total error including both the distance error and the direction error.

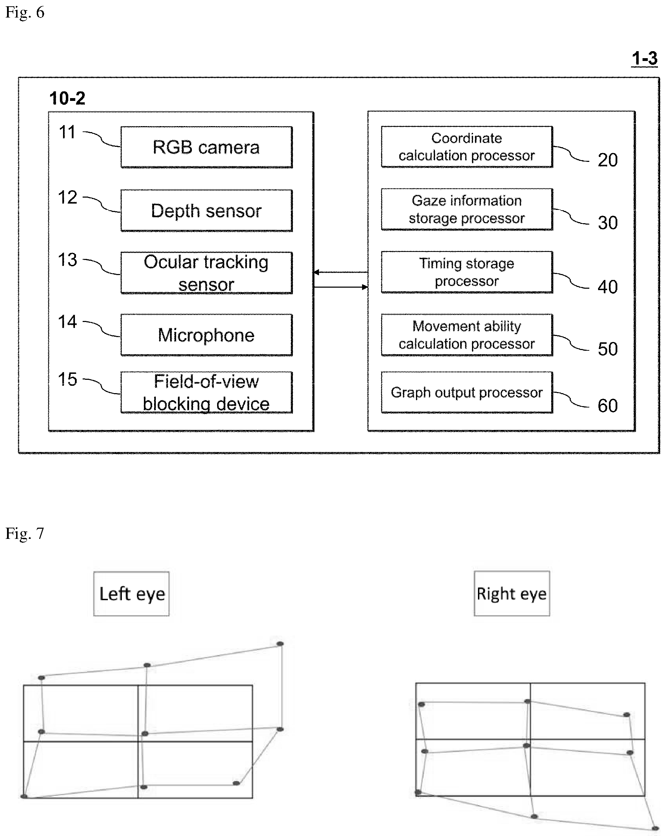

FIG. 6 is a block diagram of an ocular movement ability information generation apparatus according to still another exemplary embodiment of the present specification.

Referring to FIG. 6, an ocular movement ability information generation apparatus 1-3 according to still another exemplary embodiment of the present specification may include a housing 10-2 including an RGB camera 11, a depth sensor 12, an ocular tracking sensor 13, a microphone 14, and a field-of-view blocking device 15, a coordinate calculation processor 20, a gaze information storage processor 30, a timing storage processor 40, a movement ability calculation processor 50, and a graph output processor 60. The RGB camera 11, the depth sensor 12, the ocular tracking sensor 13, the microphone 14, the field-of-view blocking device 15, the coordinate calculation processor 20, the gaze information storage processor 30, the timing storage processor 40, and the movement ability calculation processor 50 have been described above so that a redundant description will be omitted.

The graph output processor 60 may output a graph for a position of the gaze of the subject according to each grid point.

FIG. 7 is an exemplary image of a graph according to gaze positions of a left eye and a right eye of a subject.

Referring to FIG. 7, the graph output processor 60 may output a graph for the position of the gaze according to each grid point for a left eye and a right eye of the subject. A graph for the left eye may mean that a function of an inferior oblique muscle of the left eye is hyperactive. A graph for the right eye may mean that a function of an inferior rectus muscle of the right eye is hyperactive.

The housing 10, 10-1, or 10-2 may further include an inertial measurement unit sensor and/or a speaker. If the subject who wears the housing 10, 10-1, or 10-2 moves a head beyond a predetermined angle range, the speaker may output a guidance voice to the subject so that the angle of the head falls within the range. Alternatively, if the angle of the head of the subject is out of the range, a warning sound is generated and the tester may verbally command the subject to locate the angle of the head within the range. Further, a guidance message may be output to the subject through the housing 10, 10-1, or 10-2 to locate the angle of the head within the range.

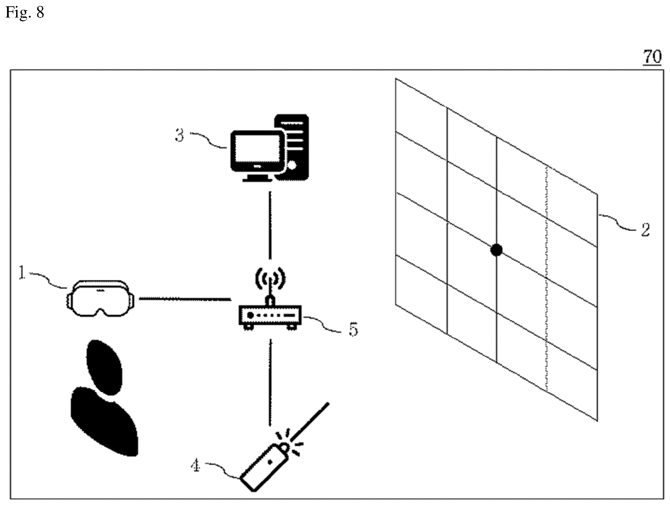

FIG. 8 is a schematic image of an ocular movement ability information generation system according to an exemplary embodiment of the present specification.

Referring to FIG. 8, the ocular movement ability information generation apparatus 1, 1-1, 1-2, or 1-3 may be a part of an ocular movement ability information generation system 70 including the indicator 2, a computing device 3, a target generation device 4, and a communication device 5. The computing device 3 may be a computer, a smartphone, and/or a tablet computer, but is not limited by specific equipment.

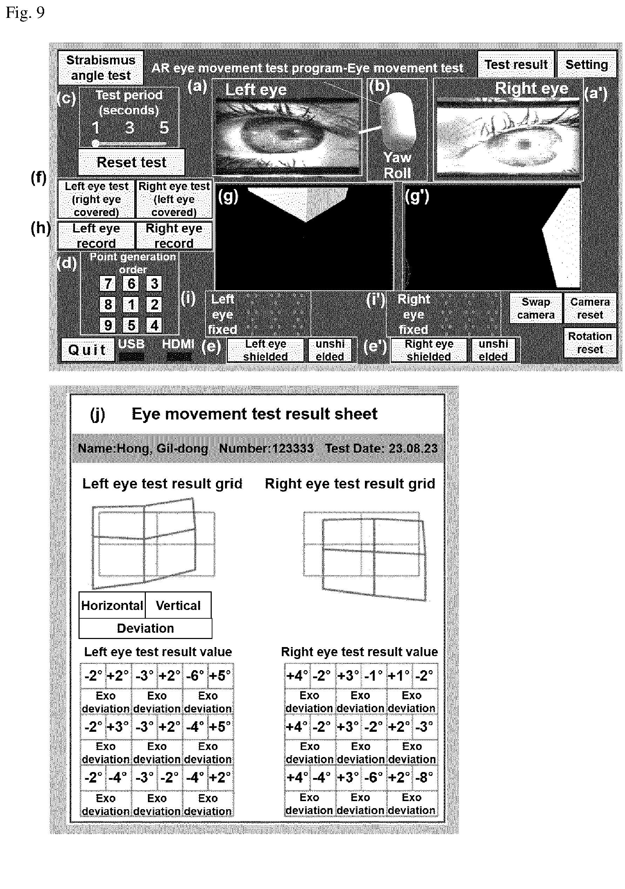

FIG. 9 is an exemplary image illustrating linkage between an ocular movement ability information generation apparatus and a computing device.

Referring to FIG. 9, the ocular movement ability information generation apparatus 1, 1-1, 1-2, or 1-3 may be controlled using software installed in the computing device 3. The computing device 3 may output an actuation signal to the ocular movement ability information generation apparatus 1, 1-1, 1-2, or 1-3 through the communication device 5. Further, the computing device 3 may receive data generated in the ocular movement ability information generation apparatus 1, 1-1, 1-2, or 1-3 through the communication device 5.

The computing device 3 may output image data of a left eye ((a) in FIG. 9) and a right eye ((a') in FIG. 9) of the subject captured by the ocular tracking sensor 13. Further, the computing device 3 may output an angle ((b) in FIG. 9) of the head of the subject measured by the inertial measurement sensor.

The computing device 3 may set a test period of the ocular movement ability information generation apparatus 1, 1-1, 1-2, or 1-3 ((c) of FIG. 9). The test period may be a time when a target is presented at each grid point. The computing device 3 may output data for which the period is set to the target generation device 4 through the communication device 5. The target generation device 4 may generate a target in the position of each grid point according to the period.

The computing device 3 may display an order of generating a target at each grid point ((d) in FIG. 9). Further, the computing device 3 may determine an order of generating the target at each grid point. Furthermore, the computing device 3 may output the data for which the order is set to the target generation device 4. The target generation device 4 may generate a target in each grid point according to the order set for the data.

The computing device 3 may output a signal to the gaze information storage processor 30 to shield or unshield the field of view of the left eye of the subject ((e) of FIG. 9). The gaze information storage processor 30 may output the signal to the field-of-view blocking device 15.

The computing device 3 may output a signal to the gaze information storage processor 30 to shield or unshield the field of view of the right eye of the subject ((e') of FIG. 9). The gaze information storage processor 30 may output the signal to the field-of-view blocking device 15.

If the field-of-view blocking device 15 shields a field of view of the left eye or the right eye of the subject, the computing device 3 may output a signal to the ocular movement ability information generation apparatus 1, 1-1, 1-2, or 1-3 to conduct the ocular movement ability test of the left eye or the right eye ((f) of FIG. 9).

During the process of generating ocular movement ability information, the computing device 3 may receive image data of the indicator 2 from the coordinate measurement processor 20 and a measurement value for the gaze direction from the gaze information storage processor 30. The computing device 3 may display an image of the indicator 2 and information about the gaze direction as an image. In (g) of FIG. 9, the image of the indicator 2 and the gaze estimation position for the measurement value measured during the left eye test of the subject may be displayed. In (g') of FIG. 9, the image of the indicator 2 and the gaze estimation position for the measurement value measured during the right eye test of the subject may be displayed. When the ocular movement ability test for the left eye and the right eye of the subject ends, the computing device 3 may output a test end signal to the ocular movement ability information generation apparatus 1, 1-1, 1-2, or 1-3. Next, the computing device 3 may quantify the test result of the left eye or the right eye of the subject ((h) of FIG. 9).

The computing device 3 may display the test result of the left eye of the subject ((i) of FIG. 9) and the test result of the right eye ((i') of FIG. 9).

The computing device 3 may receive a graph for the gaze position of the left eye and the right eye output from the graph output processor 60. The computing device 3 may output a result sheet ((j) of FIG. 9) including a deviation value of the left eye for each grid point and/or a deviation value of the right eye for each grid point of the graph. The computing device 3 may output the result sheet as a text file and/or an image file, which corresponds to an example, but is not limited by a specific file format.

The computing device 3 may output a signal which initializes the generated ocular movement ability information, angle information of the head of the subject, setting information of the RGB camera 11, the depth sensor 12, and/or the ocular tracking sensor 13. Further, the computing device 3 may output communication state and/or test state information with the ocular movement ability information generation apparatus 1, 1-1, 1-2, or 1-3.

A linkage method between the computing device 3 and the ocular movement ability information generation apparatus 1, 1-1, 1-2, or 1-3 corresponds to an example and is not limited by the above-described method.

In the present specification, it was assumed that the ocular movement ability test of the subject was conducted through the Hess screen chart test. However, it is obvious that the present specification is not limited to the Hess screen chart test and there may be various exemplary embodiments depending on various types of ocular movement ability tests, such as the Lancaster red-green test.

The coordinate calculation processor 20, the gaze information storage processor 30, the timing storage processor 40, the movement ability calculation processor 50, and the graph output processor 60 may include processors, application-specific integrated circuits, other chipsets, logic circuits, registers, communication modems, and data processing devices which are known in the technical field to which the present disclosure belongs to execute the calculation and various control logics. Further, when the above-described control logic is implemented as software, the coordinate calculation processor 20, the gaze information storage processor 30, the timing storage processor 40, the movement ability calculation processor 50, and the graph output processor 60 may be implemented by a set of program modules. At this time, the program module may be stored in the memory device and executed by the processor.

Hereinafter, an ocular movement ability generation method using the ocular movement ability information generation apparatuses 1, 1-1, 1-2, or 1-3 according to the present specification will be described. However, repeated descriptions for each component will be omitted in the description of the ocular movement ability generation method according to the present specification.

The ocular movement ability information generation method according to the present specification is an ocular movement ability information generation method using an apparatus including a predetermined shape of housing 10 which is mounted on a head of the subject and includes an RGB camera 11, a depth sensor 12, and an ocular tracking sensor 13.

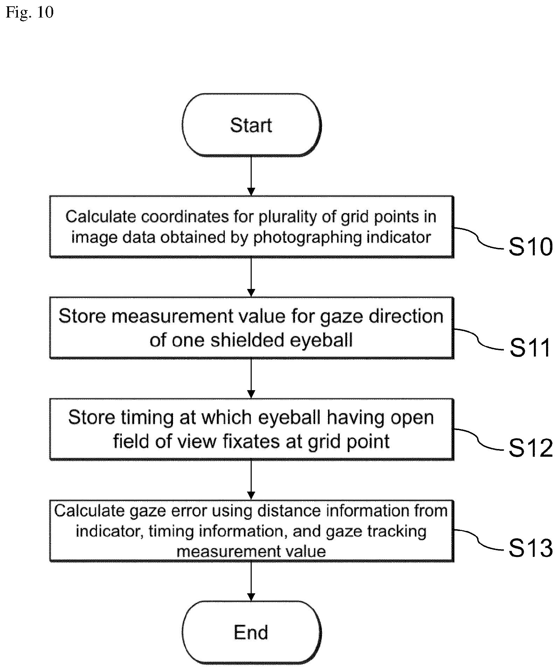

FIG. 10 is a flowchart of an ocular movement ability information generation method according to an exemplary embodiment of the present specification.

Referring to FIG. 10, in step S10, when a subject wearing the housing 10 fixates at an indicator 2, the processor may receive image obtained by photographing the indicator from the RGB camera 11. The processor may calculate a coordinate for a plurality of grid points in the image data. In step S11, when the subject fixates at the indicator while blocking a field of view of one of two eyes, the processor may receive and store a measurement value for a gaze direction of an eyeball having a shielded field of view from the ocular tracking sensor 13. In step S12, when the subject fixates at a target presented in each grid point, the processor may store timing information for the fixate at each grid point. In step S13, the processor may receive distance information between the subject and the indicator 2 from the depth sensor 12. The processor may calculate a gaze error of the subject for each grid point using the distance information, the timing information, and the gaze tracking measurement value.

In step S13, the processor may calculate a mean value using the gaze tracking measurement value stored at the timing when the subject fixates at the grid point. The processor may calculate an estimated position on the indicator 2 using the mean value. The processor may calculate a distance error between the estimated position and each grid point. Further, the processor may calculate a direction error for each grid point using the distance information and the estimated position. Further, the processor may calculate a total error including both the distance error and the direction error.

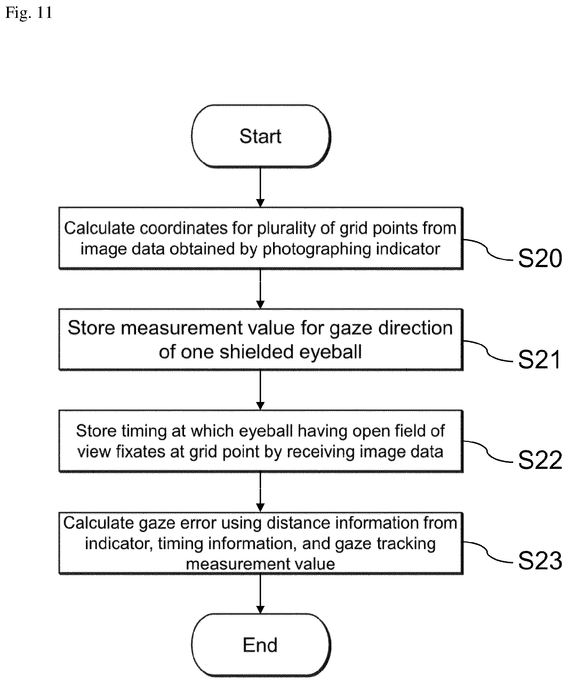

FIG. 11 is a flowchart of an ocular movement ability information generation method according to another exemplary embodiment of the present specification.

Referring to FIG. 11, steps S20, S21, and S23 are the same as the steps S10, S11, and S13 so that a redundant description will be omitted.

In step S22, the processor may receive image data in which a target is presented at each grid point from the RGB camera 11. The processor may store timing information according to the target presented in each grid point position.

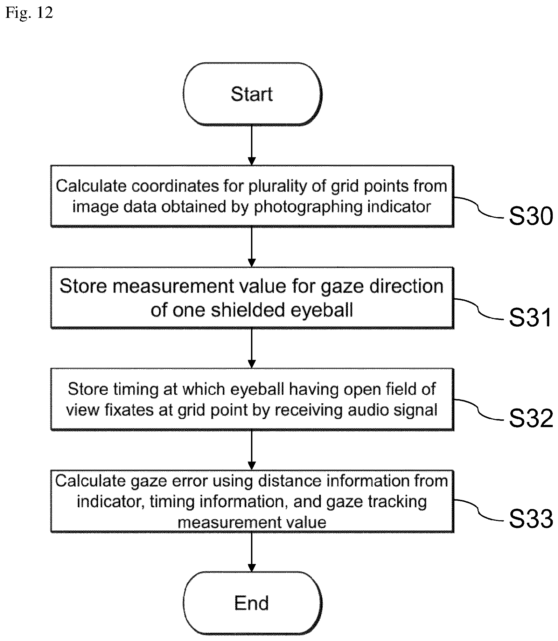

FIG. 12 is a flowchart of an ocular movement ability information generation method according to still another exemplary embodiment of the present specification.

Referring to FIG. 12, steps S30, S31, and S33 are the same as the steps S20, S21, and S23 so that a redundant description will be omitted.

The housing 10-1 according to another exemplary embodiment of the present specification may further include a microphone 14 which receives an audio signal. The processor may receive an audio signal from the microphone 14. The audio signal may correspond to a voice signal of the tester and/or a beep sound. If the audio signal is input, the processor may store a timing when the subject fixates at each grid point.

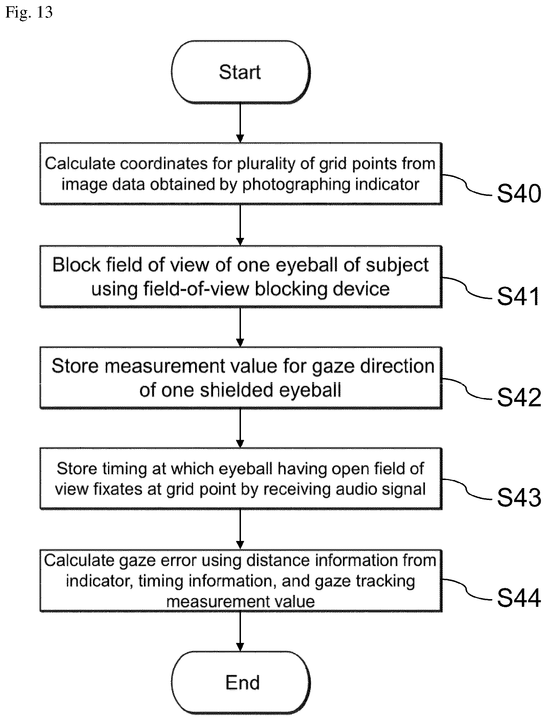

FIG. 13 is a flowchart of an ocular movement ability information generation method according to still another exemplary embodiment of the present specification.

Referring to FIG. 13, steps S40, and S42 to S44 are the same as the steps S30 to S33 so that a redundant description will be omitted.

The housing 10-2 according to still another exemplary embodiment of the present specification may further include a field-of-view blocking device 15 which individually blocks a field of view of the subject. In step S41, the processor may output an actuation signal to the field-of-view blocking device 15 to block a field of view of one eyeball of the subject.

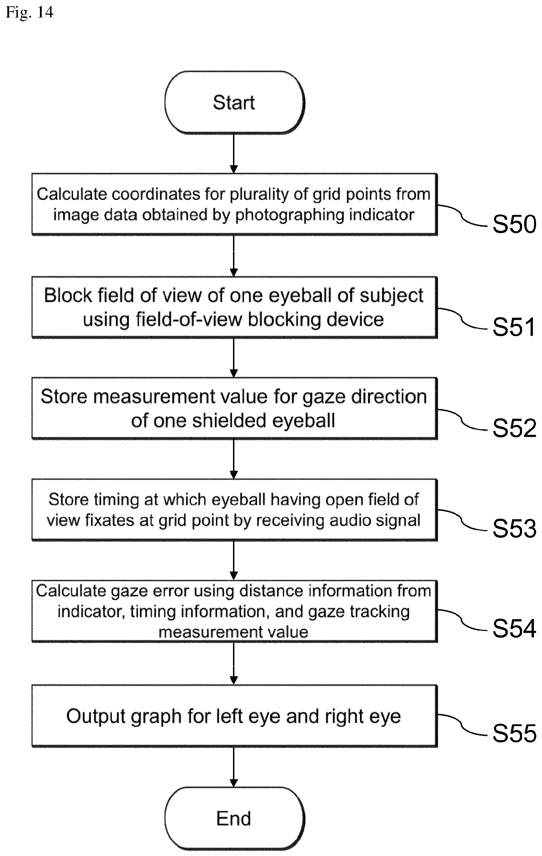

FIG. 14 is a flowchart of an ocular movement ability information generation method according to still another exemplary embodiment of the present specification.

Referring to FIG. 14, steps S50 to S54 are the same as the steps S40 to S44, so that a redundant description will be omitted.

In step S55, the processor may output information on the gaze for the left eye and the right eye of the subject as a graph.

The ocular movement ability information generation method according to the present specification may be implemented as a computer program which is created to perform each step in the computer to be recorded in a computer readable recording medium. The above-described computer program may include a code which is coded by a computer language, such as C/C++, C#, JAVA, Python, or machine language which can be read by the processor (CPU) of the computer through a device interface of the computer to allow the computer to read the program and execute the method implemented by a program. Such a code may include a functional code related to a function which defines functions required to execute the above-described methods and include an execution procedure-related control code required for the processor of the computer to execute the functions according to a predetermined procedure. Such a code may further include a memory reference-related code indicating a location (address) of an internal or external memory of the computer where additional information or media required to allow the processor of the computer to execute the above-mentioned functions is referenced. Further, if the processor of the computer requires communication with any other remote computer or server to execute the functions, the code may further include a communication-related code about how to communicate with any other remote computer or server using a communication module of the computer and which information or media needs to be transmitted or received during the communication.

The storage medium refers to a medium that stores data semi-permanently and is readable by the device, rather than a medium that stores data for a short moment, such as registers, caches, and memories. Specifically, examples of the storage medium include a ROM, a RAM, a CD-ROM, a magnetic tape, a floppy disk, or an optical data storage device, but are not limited thereto. That is, the program may be stored in various recording media on various servers accessible by the computer or in various recording media on the computer of the user. Further, the media may be distributed to computer systems connected through a network, so that a computer readable code may be stored and executed by a distribution method.

The exemplary embodiments of the present disclosure have been described with reference to the accompanying drawings, but those skilled in the art will understand that the present specification may be implemented in another specific form without changing the technical spirit or an essential feature thereof. Therefore, it should be understood that the above-described exemplary embodiments are illustrative in all aspects and do not limit the present specification.

### [Explanation of Reference Numerals and Symbols]

1: Ocular movement ability information generation apparatus
2: Indicator
10: Housing
11: RGB camera
12: Depth sensor
13: Ocular tracking sensor
14: Microphone
15: Field-of-view blocking device
20: Coordinate calculation processor
30: Gaze information storage processor
40: Timing storage processor
50: Movement ability calculation processor
60: Graph output processor
70: Ocular movement ability information generation system

## Claims

1. A method for generating ocular movement ability information using a device including an RGB camera, a depth sensor, and an ocular tracking sensor in a housing of a predetermined shape mounted on a head of a subject, the method comprising:
(a) calculating, by a processor, coordinates for a plurality of grid points in image data obtained by photographing an ocular movement indicator input from the RGB camera;
(b) receiving, from the ocular tracking sensor, by the processor, a measurement value for a gaze direction of one of two eyes of the subject of which a field of view is blocked, and storing the measurement value;
(c) storing, by the processor, timing (hereinafter, referred to as "timing information") at which one of the two eyes of the subject of which the field of view is open fixates at the grid points included in the ocular movement indicator; and
(d) calculating, by the processor, a gaze error of the subject with respect to each of the grid points by using distance information between the subject and the indicator input from the depth sensor, the timing information, and the gaze tracking information.

2. The method for generating ocular movement ability information of claim 1, wherein the housing further includes a microphone which receives an audio signal, and
wherein the (c) is, receiving, by the processor, the audio signal from the microphone or information of a target image presented at the grid point from the RGB camera to store the timing information.

3. The method for generating ocular movement ability information of claim 1, wherein the housing further includes a field-of-view blocking device which individually blocks fields of view of the two eyes of the subject and
wherein the (b), the processor outputs a blocking signal to the field-of-view blocking device to block the field of view of one eye of the subject.

4. The method for generating ocular movement ability information of claim 1, wherein the (d) is, quantifying, by the processor, at least one of an average distance error between gaze positions of the subject in the grid points, a direction error for each grid point, and a total error.

5. The method for generating ocular movement ability information of claim 1, further comprising:
after the (d),
(e) outputting, by the processor, a graph for a position of a gaze of the subject according to each grid point.

6. A computer program which is created to allow a computer to perform each step of the method for generating ocular movement ability information according to any one of claims 1 to 5 to be recorded in a computer readable recording medium.

7. An apparatus for generating ocular movement ability information, comprising:
a housing of a predetermined shape which is mounted on a head of a subject and includes an RGB camera, a depth sensor, and an ocular tracking sensor;
a coordinate calculation processor which calculates coordinates for a plurality of grid points in image data obtained by photographing an ocular movement indicator input from the RGB camera;
a gaze information storage processor which receives a measurement value for a gaze direction of one of two eyes of the subject of which the field of view is blocked and stores the measurement value;
a timing storage processor which stores timing (hereinafter, referred to as "timing information") at which one of the two eyes of the subject of which the field of view is open fixates at the grid points included in the ocular movement indicator; and
a movement ability calculation processor which calculates a gaze error of the subject with respect to each of the grid points by using distance information between the subject and the indicator input from the depth sensor, the timing information, and the gaze tracking information.

8. The apparatus for generating ocular movement ability information of claim 7, wherein the housing further includes a microphone which receives an audio signal, and
the timing storage processor receives the audio signal from the microphone or information of a target image presented at the grid point from the RGB camera to store the timing information.

9. The apparatus for generating ocular movement ability information of claim 7, wherein the housing further includes a field-of-view blocking device which individually blocks fields of view of the two eyes of the subject and
the gaze information storage processor outputs a blocking signal to the field-of-view blocking device to block the field of view of one eye of the subject.

10. The apparatus for generating ocular movement ability information of claim 7, wherein the movement ability calculation processor quantifies at least one of an average distance error between gaze positions of the subject in the grid points, a direction error for each grid point, and an total error.

11. The apparatus for generating ocular movement ability information of claim 7, further comprising:
a graph output processor which outputs a graph for a position of a gaze of the subject according to each grid point.

12. A system for generating ocular movement ability information, comprising:
the apparatus for generating ocular movement ability information according to any one of claims 7 to 11;
an ocular movement indicator on which a plurality of grid points is drawn;
a target generation device which presents a target image on the grid point;
a computing device which outputs an actuation signal of the apparatus for generating ocular movement ability information and receives ocular movement ability information; and
a communication device which transmits and receives a signal or information between the apparatus for generating ocular movement ability information, the computing device, and the target generation device.
